# EUROPEAN PATENT SPECIFICATION

(11) **EP 1 871 454 B1**
(45) Date of publication and mention of the grant of the patent: **02.03.2011**
(21) Application number: 06706141.6
(22) Date of filing: 20.03.2006
(51) Int. Cl.: A61M 25/02, A61M 5/158

(54) **A MOUNTING PAD, AN ADHESIVE DEVICE COMPRISING SUCH MOUNTING PAD, AND METHODS OF PREPARING AN INFUSION**
ANBRINGKISSEN, KLEBEVORRICHTUNG MIT EINEM SOLCHEN ANBRINGKISSEN UND VERFAHREN ZUM VORBEREITEN EINER INFUSION
TAMPON DE MONTAGE, DISPOSITIF ADHESIF COMPRENANT UN TEL TAMPON ET PROCEDES DE PREPARER UNE PERFUSION

(30) Priority: 21.03.2005 US 663804 P; 21.03.2005 DK 200500406
(43) Date of publication of application: 02.01.2008
(73) Proprietor: Unomedical A/S, 3460 Birkerød (DK)
(72) Inventor: JENSEN, Søren, 3540 Lynge (DK); GÖRANSSON, Magnus Walter, 233 93 Svedala (SE); KORNERUP, Grete, 4390 Vipperød (DK); MOGENSEN, Lasse Wesseltoft, 2750 Ballerup (DK)
(74) Representative: Olsen, Lars Pallisgaard
(86) International application number: PCT/DK2006/050008
(87) International publication number: WO 2006/099876

(56) References cited:
- EP-A- 0 117 632
- EP-A- 0 239 244
- EP-A- 0 322 210
- EP-A- 1 407 747
- EP-A- 1 421 968
- WO-A-98/10823
- WO-A-98/15312
- WO-A-02/083206
- WO-A-2006/067217
- US-A- 5 384 174
- US-A- 5 520 629
- US-A- 5 702 371
- US-A1- 2002 026 152
- US-A1- 2002 165 493
- US-B1- 6 520 938

## Description

### Background of the invention

The present invention relates in its broadest aspect to mounting pads having a release layer for use in devices applied onto the skin of a patient, particularly infusion sets. The invention also relates to adhesive devices and particularly to adhesive devices including an injector device and an infusion set and a mounting pad having a release layer which can be automatically removed from the mounting pad when the infusion set is applied to a patient. The invention furthermore relates to methods of applying infusion sets to a patient.

### Description of the related art

Products in the form of an adhesive sheet material for use on the skin, comprising a release layer are well known. The release layer can be divided into several sections which can be removed manually from the adhesive device independently of each other. A product in the form of infusion sets comprising a mounting pad and a release layer are also well known. The release layer can e.g. be manually removed in one piece from the plaster before applying the infusion set on the patient.

WO02/083206 relates to an infusion set which is attached to the skin of a wearer. US 7702371, US 2002/0165493, US 2002/0026152 and EP 1 421 968 relate to anchoring medical devices such as catheters to a patient. EP 0 117 632 provides a thin adhesive coated film dressing, and EP 1 407 747 relates to an absorbent article.

Such types of products require manual removal of the release layer before or after the medical device set is applied to the patient. In some cases when the release layer is removed manually before the infusion set is applied to the patient, the adhesive layer can fold, become contaminated or be inappropriately attached to the patient. When the release layer is removed after the infusion set has been applied to the patient the removal of the release layer can cause difficulties and discomfort when fastening the infusion set to the patient, as the infusion set is not secured to the user when the release layer is removed, although the cannula is already inserted into the patient. The release layer is often removed in one piece.

With the known products it can also be difficult to remove all of the release layer, especially in the cases where the release layer is in one piece.

A product in the form of a system for delivering adhesive strips and transparent dressings to a patient, especially very thin, adhesive-coated transparent films are known. Such a system comprises a multi-layer system, where the removal of one layer exposes an adhesive surface of the underlying layer. A support layer carrying the underlying layer can then e.g. be used for applying the underlying layer on the skin and the support layer can subsequently be removed.

It is also known to sell bandage in packages where the package constitute at least a part of the release layer. The bandage in this form can include a release layer which is fastened to the package. The bandage is applied to a wound by manually opening the package by pulling flaps and thereafter by continuing the pulling motion applying the bandage to the wound until the bandage is attached to the skin.

However, these systems are not fully automatic, and do not relieve the user of detailed and fine finger movements being problematic for people with bad dexterity. Neither does the system facilitate applying devices or parts of devices onto a patient. These systems are furthermore not rapid, which might be desirable in some instances.

Diabetes is a disease which requires constant observation of the glucose level in the patient's blood and injections of insulin. To control and facilitate the treatment some diabetics use an insulin pump connected to an infusion device for subcutaneous delivery into the patient, often through a cannula. On certain occasions, e.g. when taking a bath the pump can be disconnected from the infusion device and reconnected later while the infusion device is left in place on the patient. The subcutaneous application of the infusion device can sometimes be difficult and painful for some diabetics for which the application device according to the present invention can be helpful.

Accordingly, it is an object of the invention to provide an adhesive device which is rapid and easy to apply to a patient.

It is another object of the invention to provide an adhesive device with automatic removal of the release layer.

It is a further object of the invention to provide a rapid and easy method of applying an infusion set to a patient.

It is still a further object of the invention to provide a method of applying an infusion set to a patient without contaminating or damaging the adhering effect of the adhesive device.

### Summary of the invention

The present invention relates in one aspect to as defined in claim 1.

According to a second aspect the present invention relates to a method as defined in claim 24.

The second and third sections of the release layer are removed together with the application device.

This is a method where the release layer is automatically removed during application. In a preferred embodiment the infusion set is delivered with the infusion part placed in the infusion set and the spring in a set condition and the projecting parts of the release layer secured to the housing of the injector device. Thus, it is only necessary to place the set at the desired site, actuate the injector device and to remove the first section of the release layer.

According to a third aspect the present invention relates to a method as defined in claim 25.

This is a method where the release layer is manually removed in steps, where one section of the release layer is first removed, thereafter the mounting pad and infusion part are applied onto the patient and finally the third and first sections of the release layer are manually removed. In a preferred embodiment of this method the infusion set is delivered with the infusion part placed in the injector device and the spring in a set condition. Then, the first step in the method will be to remove a second section of the release layer.

According to a seventh aspect the present invention relates to a kit comprising a mounting pad, an infusion part and an injector device.

The present invention relates to a mounting pad as defined in claim1.

These mounting pads may be used alone as a plaster or a dressing such as a wound dressing or preferably together with devices, for applying infusion sets or parts of infusion sets, e.g. an infusion part, onto a patient.

According to a preferred embodiment the projecting parts comprising a first projecting part defining a central axis of the mounting pad and at least two secondary projecting parts are essentially parallel.

According to a preferred embodiment the secondary projecting parts are positioned symmetrical around the central axis defined by the first projecting part.

According to another preferred embodiment, the release layer is divided into two or more sections.

According to yet another preferred embodiment, the release layer is divided into at least two sections, a first section being provided with a first part projecting along the essentially central axis of the mounting pad, and two projecting parts, one on each side of first part, and a remaining section being provided with two projecting parts.

According to a further embodiment, the two projecting parts of the remaining section overlap with the two projecting parts of the first section.

According to another preferred embodiment, the remaining section of the release layer is divided into two sections, a second and a third section.

According to a further preferred embodiment, the remaining section of the release layer is divided into a second and third section along the central axis of the mounting pad.

According to yet another preferred embodiment, the remaining section is divided perpendicularly to the central axis of the mounting pad.

According to another preferred embodiment, the sections of the release layer can be removed from the mounting pad independently from each other. Preferably the mounting pad comprises a hole located along essentially a central axis of the mounting pad allowing passage of a cannula to be inserted into the skin.

According to a preferred embodiment the mounting pad has an adhesive surface being provided with a release layer, said release layer being divided into three sections perpendicularly to the central axis of the mounting pad, a first section having a first part projecting along an essentially central axis of the mounting pad and two projecting parts on each side of the first part, a second section comprising two projecting parts, and a third section, wherein the projecting parts of the of the first section are essentially parallel to and spaced apart from the first part and the projecting parts of the second section are superposed on the two projecting parts of the first section when the sections are arranged together on the adhesive surface.

According to another preferred embodiment, the mounting pad has an adhesive surface being provided with a release layer, said release layer being divided into three sections, a first section having a first part projecting along an essentially central axis of the mounting pad and two projecting parts on each side of the first part, said first section being placed on the front part of the adhesive surface between the front part of the adhesive surface and the second and third sections, the second and third sections each comprising one projecting part, said second and third sections being separated along the central axis of the mounting pad, and the projecting parts of the second and third sections of the release layer being superposed on the projecting parts of the first section.

The present invention relates to an adhesive device comprising an application device and a mounting pad having an adhesive surface, said surface being provided with a release layer, wherein the mounting pad is displaceable in relation to the housing of the application device during application, and wherein the release layer is secured to the housing.

The invention is based on the discovery that by securing a part of a release layer of a mounting pad to an application device the peeling of the release layer off the mounting pad may be effected automatically when the mounting pad is displaced in relation to the application device during application. This secures a clean adhesive surface of the mounting pad which results in optimal adhesion of the mounting pad to the user.

According to another embodiment the invention relates to an adhesive device, wherein at least one projecting part of the first, second or third section is secured to the housing of the application device.

According to a further embodiment the invention relates to an injector device wherein the mounting pad is longitudinally displaceable from a retracted position in relation to the application device by which displacement a spring is set, said device comprising means for retaining the mounting pad in that position, said device further comprising means for actuating the spring releasing the mounting pad. Accordingly, the actuation of the spring of the application device secures rapid application of the application device on the skin of the user, without the need for manual intervention.

This is highly relevant e.g. for diabetics which have to, or may desire to insert an infusion device or to place a subcutaneous sensor or the like on themselves. For some persons it is a troublesome and uncomfortable process to perform the skin penetration themselves and they therefore need a device which assists them in this process, thereby making the process less problematic. It is also relevant for older persons or persons with weak fingers or limited dexterity to be able to apply such devices on themselves. In those cases the adhesive devices of the present invention assist by providing an automatic and rapid application of a device on the user's body which does not require fine finger movements.

According to one embodiment of the present invention the release layer of the mounting pad is divided into two or more sections. According to another embodiment at least one projecting part of the first, second or third section is secured to the housing of the application device.

The adhesive component is preferably a skin friendly adhesive known per se, provided with a micro porous backing, one side of the component having an adhesive surface.

The adhesive surface of a mounting pad is provided with a release layer for protecting the adhesive surface until use. The release layer is preferably made from siliconized paper or from a sheet of polyethylene which can easily be removed from the adhesive surface without damaging the same.

According to a preferred embodiment of the invention the release layer provided with a non-touch feature known per se enabling an easy manipulation of the release layer without risk of touching the adhesive surface, especially in case of an embodiment for manual removal of the release layer.

According to another preferred embodiment of the invention the mounting pad of the adhesive device includes an infusion part of an infusion set.

According to yet another preferred embodiment of the invention the application device of the adhesive device is an injector device.

The adhesive device comprises an application device and an infusion set or a part of an infusion set comprising an infusion part for insertion into a patient, said infusion part comprising an mounting pad and a release layer covering at least a part of the mounting pad, said infusion set being displaceable in relation to the application device, wherein at least a part of the release layer is connected to the application device.

In a preferred embodiment the infusion part comprises a base part with a first set of guiding means, a first cannula extending from said base part and being in fluid communication with a cavity which is optionally covered with a membrane, said cavity being further adapted to receive a second cannula extending from a connector, a second set of guiding means adapted to fit with the first set of guiding means and at least two arms, said retention devices extending from an upper surface of the main surface of the base part, further comprising an injector device, said release layer being secured to the injector device.

In a second aspect the invention relates to a method as defined in claim 24.

This method ensures a rapid and secure application of an infusion part to a patient in an easy manner, providing an optimum placement of a cannula included in the infusion part without causing too much pain to the user. The method furthermore minimizes contamination of the adhesive surface of the mounting pad, as the release layer is removed automatically during the application.

An illustrative example renders it possible to manually utilise the mounting pad of the invention together with an application device. In this manner, a part of the release layer is removed manually before the injector device is actuated, and after the adherence of the corresponding part of the mounting pad, the projections are utilised for manually pressing the pad against the skin while manually removing the remaining parts of the release layer and pressing the adhesive surface against the skin.

When the mounting pad includes a release layer which is automatically removed, the device functions accordingly. An infusion part on a mounting pad is inserted into the application device. The two projecting parts of the second and third sections of the release layer are connected to the housing of the application device. They can be secured by pressing a button through the holes of the projecting parts and corresponding holes on the application device or e.g. by welding or gluing them to the device. Thereafter first projecting part - also called the tongue - of the first section of the release layer is secured to the pivoting member of the application device. Preferably the tongue is inserted through a hole in the pivoting member, the tongue having an enlarged surface area at the end providing a locking effect. This combination of the infusion part and the mounting pad in the application device can also be the starting point for the user. Now the application device is in a locked state, as the pivoting arm is placed parallel to the housing of the application device and the longitudinal movement of said pivoting arm along the housing is hindered by the first locking means. By pulling the pivoting member of the application device and placing it in a position perpendicular to the housing said device is ready for use. Now a front end of the application device is held in contact with the skin, preferably approximately perpendicularly to the skin, and the device is thereafter activated by pressing the release means. When the device is actuated the infusion part on the mounting pad is pushed in the direction towards the skin and during this movement the second and third section of the release layer will start to peel off the mounting pad, as the two projecting parts and the tongue are secured to the application device, preferably on different sides of the housing of the device. Accordingly, the first section of the release layer and the mounting pad will move in one direction while the second and third section of the release layer will begin to move in a deviating direction, thus gradually exposing the adhesive surface of the mounting pad. When the mounting pad gets in contact with the skin of the user the adhesive surface is exposed and adheres to the skin and the cannula of the infusion part is placed subcutaneously in the patient with the help of the needle of the application device. The tongue of the first section is released from the pivoting member and the application device is lifted away from the skin. During the removal of the application device the two projecting parts of the first section are pressed down using two fingers in order to hold the mounting pad in place and to ease the pressing of the rest of the mounting pad to the skin for it to adhere correctly. The needle is removed from the cannula of the infusion part and the second and third sections of the release layer are completely removed from the mounting pad. The first section is removed by pulling the tongue. The mounting pad is now secured flat on the skin of the patient and the cannula of the infusion part is subcutaneously inserted into the patient. Finally for safety reasons the needle of the application device is destroyed by folding the pivoting member over the needle and by securing the pivoting member the third locking means.

In a preferred embodiment the pivoting member is swung from the position essentially orthogonal to a main axis of the application device, 180 degrees to a second position embracing and securing the needle said position also being essentially orthogonal to said main axis. Optionally the needle is destroyed in the process and secured in the pivoting member.

According to a preferred embodiment the invention relates to a method of applying an infusion set to a patient, comprising
providing an infusion part having a mounting pad provided with a release layer
removing the second part of the release layer
applying the mounting pad with the infusion part onto a patient
removing the third section of the release layer, and
removing the first section of the release layer.

In accordance with a third aspect the invention relates to a method as defined in claim 25.

When the mounting pad includes a release layer for manual removal the device functions in a similar manner as described above until the device is activated. When the pivoting member is pulled back, the second section of the release layer is exposed and subsequently peeled off by pulling one of the projecting parts exposing the adhesive surface underneath. Now the application device is held in contact with the skin, preferably perpendicularly to the skin, and the device is thereafter activated by pressing the release means. When the device is actuated the infusion part on the mounting pad is pushed in the direction to the skin. When the mounting pad gets in contact with the skin of the user the exposed adhesive surface adheres to the skin. The cannula of the infusion part is now subcutaneously inserted into the patient with the help of the needle in the application device. The centrally projecting part of the first section is released from the pivoting member and the application device is lifted away from the skin. During the removal of the application device the two projecting parts of the first section are pressed down using the fingers in order to hold the mounting pad in place. At the same time one of the projecting parts of the third section of the release layer are pulled to remove the third section. The two projecting parts of the first section also ease the pressing of the rest of the mounting pad to the skin in order for it to adhere to in correctly. The needle is removed from the cannula of the infusion part during the removal of the application device. The first section is removed by pulling the projecting part. The mounting pad is now secured flat on the skin of the patient and the cannula of the infusion part is subcutaneously inserted into the patient. Finally for safety reasons the needle of the application device is destroyed by folding the pivoting member over the needle and by securing the pivoting member the third locking means.

The invention also relates to a kit comprising an mounting pad, in combination with an infusion part and an injector device, as defined in claim 27.

### Definitions:

The expression "mounting pad" is used in the present context to designate an adhesive component having a backing layer and an adhesive layer having an adhesive surface for securing a medical device such as a plaster, a wound dressing, an ostomy appliance, or an infusion set to the skin of a patient.

The expression "release layer" is used in the present context to designate a protective layer, which protects the adhesive surface of the mounting pad before use. The release layer covers essentially the entire adhesive surface and can extend beyond the boundaries of the mounting pad forming extensions or projecting parts extending beyond the boundaries of the mounting pad. The extensions/projecting parts facilitate gripping the release layer for removal thereof or securing the same to an application device. The expression "projecting part" is used in the present context to designate a part of a release layer which extends beyond the boundary of the adhesive surface it protects. A "projecting part" can either be made of the same material and/or layer as the release layer or be a separate unit attached to the release layer.

The expression "adhesive device" is used in the present context to designate a medical device such as a plaster, a wound dressing, an ostomy appliance, or an infusion set having an adhesive surface for adhering to the skin of a patient.

The expression "application device" is used generally to designate a device for the application of an adhesive device onto the skin of a patient, or for the subcutaneous introduction of e.g. a cannula and/or a needle into the skin of a patient.

The expression "injector device" is used in the present context to designate a device for the subcutaneous introduction of the cannula of the infusion part of an infusion set into the skin of a patient.

The expression "infusion set" is used in the present context to designate a set comprising an infusion part provided with a cannula to penetrate the skin of a person and a connector for connecting the infusion part with a medical device preferably a medical delivery device such as an insulin pump. An infusion set has in its assembled form a substantially planar rear side and a relatively large width compared to its thickness, thus allowing it to lie flat on the patient's skin and thereby minimizing the discomfort of carrying the infusion set. The infusion part is placed in the patient for a longer and not specified time period while the connector can be connected and disconnected from time to time. Hereby it is possible for the patient to disconnect from the medical device, move around and at a later point reconnect to the medical device. Further it is possible to shift between different medical devices using the same infusion part thereby only one penetration of the skin is needed providing less discomfort to the patient.

The expression "infusion part" is used in the present context to designate a part of a device for subcutaneous introduction of a drug to a patient. The infusion part is provided with a cannula to penetrate the skin and is usually placed on a mounting pad for attaching the infusion part to the skin of a patient.

The expression "front part" of a release layer or an adhesive surface is used in the present context to designate a part of the release layer or adhesive surface being located nearest the site of injecting a cannula.

The expression "proximal" surface is used in the present context to designate a surface to be in contact with the users skin, and the expression "distal surface" is used to designate a surface turned away from or in a distance to the user's skin.

### Brief description of the drawings

In the following the invention will be described in further details with reference to the drawings.
Figures 1 A-E show top-view of an injector device not forming part of the invention illustrating the steps of application of an infusion part into the skin of a patient.
Figures 2 A-E show the injector device shown in fig. 1 illustrating the steps of application of an infusion part into the skin of a patient, seen from the opposite side.
Figure 3 shows an injector device not forming part of the invention located on the skin of a patient during application of an infusion part.
Figure 4 shows a top view of a part of an illustrative example of an infusion part and a mounting pad according to the invention.
Figure 5 shows a cross-sectional view along the line A-A of the infusion part shown in fig. 4.
Figure 6 shows the embodiment of an illustrative infusion part according to the invention shown in fig. 4, seen from a slanting angle.
Figure 7 shows an embodiment of the mounting pad according to the invention seen from above (distal side).
Figure 8 shows the embodiment of the mounting pad shown in fig. 7, seen from the opposite side (proximal side).
Figure 9 shows an embodiment of the first section of a release layer according to the present invention.
Figure 10 shows another example of a mounting pad seen from above (distal side).
Figure 11 shows the example of the mounting pad shown in fig. 10, seen from the opposite side (proximal side).
Figure 12 shows an injector device.

### Description of preferred embodiments

The invention is explained more in detail with reference to the drawings showing preferred embodiments of the invention.

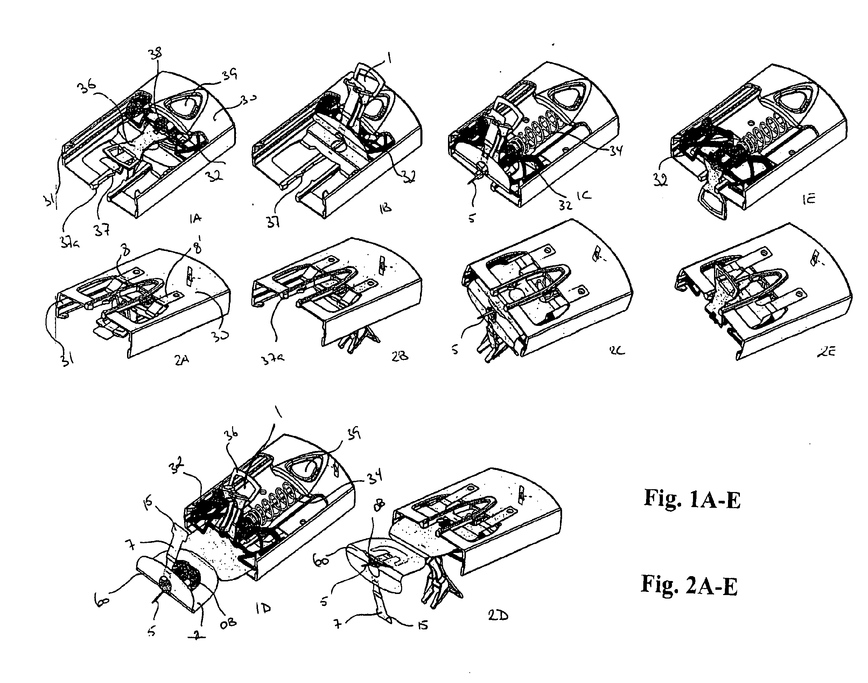

Figs. 1 A-E show an example of an injector device and illustrate the steps of application of an infusion part (0B) into a patient with the infusion part (0B) seen from above or from the distal side.

The injector device comprises a housing (30) and an infusion part (0B) provided with a mounting pad (2), which is contained in the housing (30), said injector device having a back (33) and being provided with two longitudinally extending guiding means (31) and a longitudinally slidable member (32). The device further comprises a pivoting member (36) which may engage with first means (37) for locking the pivoting member in one position where it protects the a cannula (5) of the infusion part (0B) to be introduced into a patient's skin and for preventing unintended release, and an insertion needle (35) for introducing the cannula. The pivoting member (36) has means for securing an enlarged surface area (15) of a centrally projecting part (7) of a release layer protecting an adhesive surface of the mounting pad until use. The slidable member (32) is capable of moving from a retracted position to a forward position, and is driven from the retracted position to the forward position by a spring (34). The spring is located between the slidable member (32) and the back (33) of the housing (30). The injector device further comprises second locking means (38) for locking the slidable member (32) in the retracted position when maintaining the spring in a compressed state. In this embodiment the second locking means has the form of a protrusion protruding from the slidable member (32) and being retained by a rigid part of the housing (30) placed above the slidable member (32). Also the injector comprises release means (39) for disengaging the second locking means (38), when releasing the locking means (38) the release means (39) which has the form of a button is pushed down, which pushes the locking means (38) down and then makes it possible for the locking means (38) to pass under the rigid part of the housing (30) positioned in front of the release means (39).

When the injector device is placed on the skin of a patient and the locking means is disengaged, the spring (34) drives the slidable member (32) and the infusion part (0B) to its forward position, thus introducing the cannula (5) of the infusion part into the patient by means of the needle. The release layer is provided with projections (8, 8') being secured to the housing (30), so when the slidable member (32) moves to its forward position, the release layer is automatically removed. After the introduction of the cannula the injector device can be withdrawn leaving the needle in a free position, and the infusion part is secured to the skin using the mounting pad (2). The pivoting member (36) can then be swung into a position in which it embraces the needle and protects patients or assisting persons from being stung.

The device is shown in a first, locked state (1A) in which the device is ready for use. The device is preferably delivered ready to use and in a locked state in which projections (8, 8') are secured to the housing (30). Fig. 1B shows the device after swinging the pivoting member (36) to a position where the pivoting member (36) is released of the first locking means (37) thereby unlocking the slidable member (32) and Fig. 1C shows the device after pressing the release means (39) and unlocking the second locking means (38) and the slidable member (32) has moved to its forward position inserting the needle and the cannula (5). Fig. 1D shows the device just before full disengagement from the infusion part (0B) and after withdrawal of the needle and fig. 1E shows the device swinging the pivoting member into the position embracing the needle. The pivoting member may be locked in this position by third locking means (37a).

In fig. 1B it can be seen how the second and third section of the release layer (20, 21) are peeled off the mounting pad, when the central projecting part (7) and hence first release layer is pulled back by the pivoting member (36), thereby revealing the adhesive surface (60) of the mounting pad (2).

Figs. 2 A-E show the injector device shown in fig. 1 illustrating the steps of application of an infusion part into the skin of a patient, seen from the proximal side.

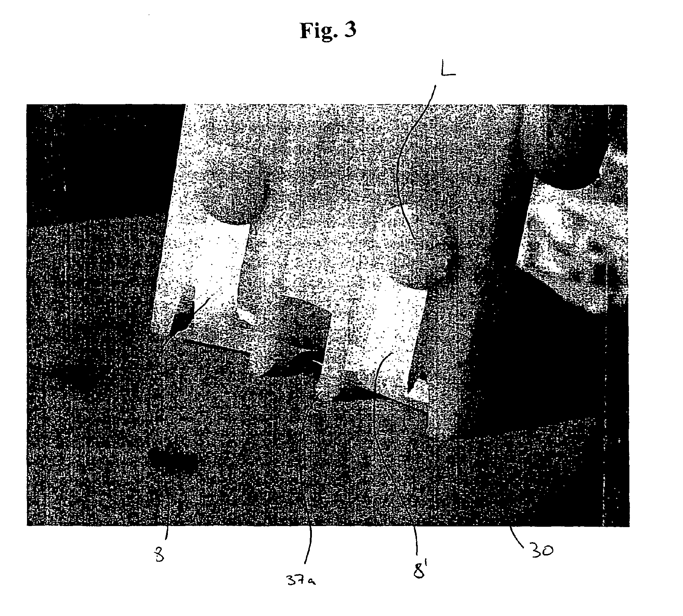

Fig. 3 shows an example of an injector device of the kind shown in Figs. 1 and 2 located on the skin of a patient during subcutaneous application of an infusion part into the patient, the device is seen from the same side as in fig. 2A-E. The device is preferably placed perpendicularly to and in contact with the skin surface before releasing the slidable member. In fig. 3 is shown the projections (8, 8') and how they are secured to the housing (30) by securing means (L) which in this embodiment has the form of buttons which are pushed into holes in the housing (30). Also the third locking means (37a) for locking the pivoting member in the position in which the pivoting member embraces the needle are shown.

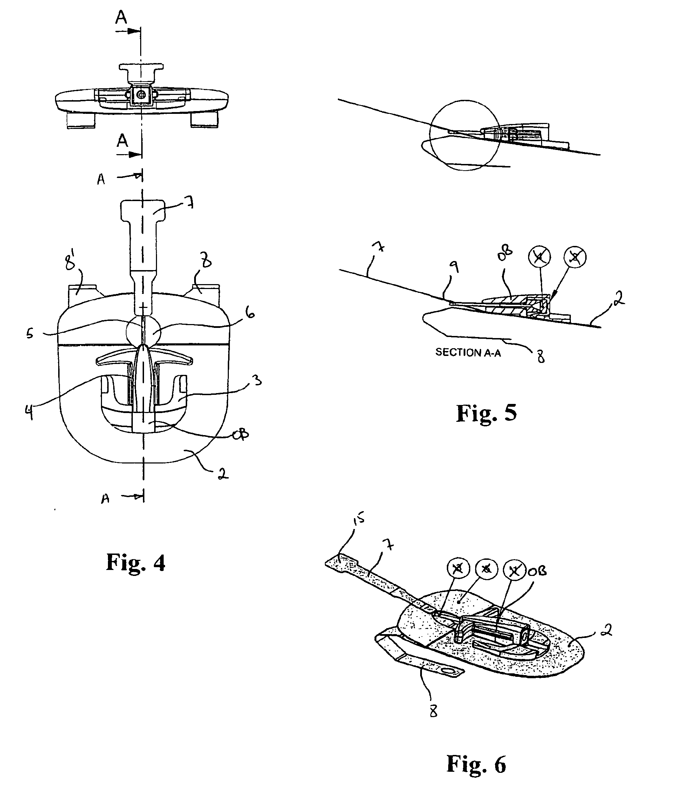

Fig. 4 shows a part of one example, an infusion part (0B) and a mounting pad (2). The infusion part comprises a base part (3) having a main plane which, when the infusion set is attached to a patient, is essentially parallel with the skin of the patient. Said base part comprises a first set of guiding means (4). Mounted on the proximal surface of the infusion part is the mounting pad (2) which in this case is a plaster. A first cannula (5), preferably a soft cannula, extends from the base part and into a hole (6) in the mounting pad (2), said cannula being in fluid communication with a cavity (not shown). The cavity optionally being covered by a membrane is adapted to receive a second cannula (not shown) extending from an external connector. The mounting pad (2) furthermore has a release layer (not shown) protecting essentially the entire adhesive surface. A central projecting part (7) extends from the mounting pad (2) and defines a central axis of the mounting pad (4). The central projecting part (7) can either be made of the same layer as the release layer or be a separate unit attached to the release layer. The mounting pad (2) furthermore comprises two additional projecting parts (8, 8') parallel to the central projecting part (7), one on each side. From this view the projecting parts (8, 8') can not be seen in full size as they are folded under the adhesive device.

Fig. 5 is a cross-sectional view of a part of the adhesive device as shown in fig. 1, along the line A-A. Shown are the infusion part (0B) and the mounting pad (2). The central projecting part (7) is connected to the first section of the release layer (9) and the other projecting parts (8, only on of the projecting parts is shown) are connected to the second or third section of the release layer and folded under the mounting pad (2).

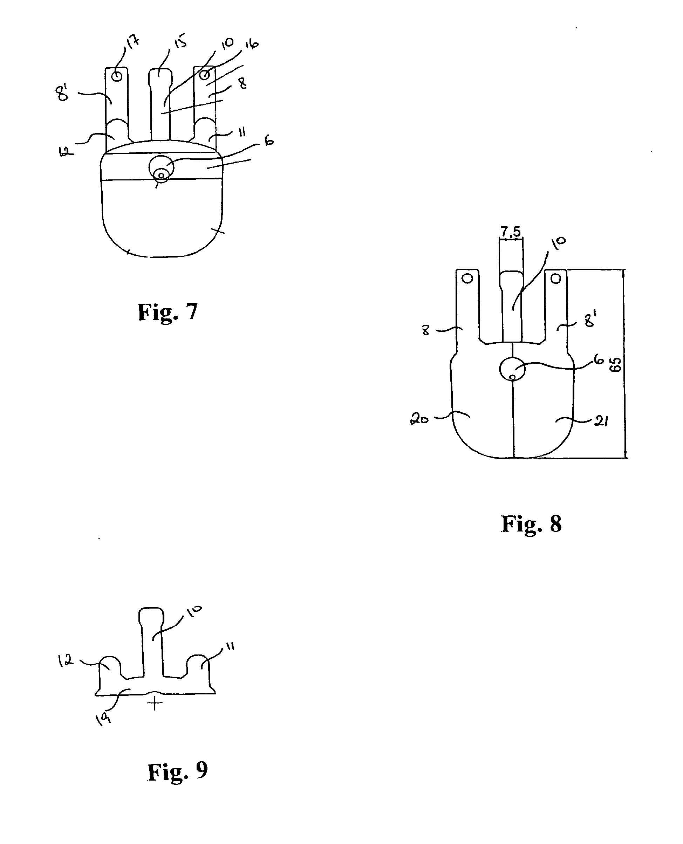

Fig. 6 shows one example of a part of the adhesive device in a state, as it is when contained in the injector device. This embodiment of mounting pad is the similar to the ones shown in figs. 7-8. The device comprises a mounting pad (2) and an infusion part (0B). The release layer comprises two projecting parts on the second and third section of the release layer (8, only one can be seen). The two projecting parts of the first section of the release layer shown in figs. 7 and 8 are not included in this embodiment. The central projecting part (7) of the first section of the release layer is temporarily fastened to the injector device during application but can be released from the injector device later, e.g. manually. The central projecting part (7) is either a part of or connected to the first section of the release layer, while the other projecting parts (8) are a part of or connected to the second or third section of the release layer.

Figs. 7-9 show the mounting pad according to the present invention. The mounting pad comprises in general an adhesive component protected with a release layer. The structure of the mounting pad shown in the figures is divided into three sections, a first, a second and a third section.

Fig. 7 shows the mounting pad in the embodiment used in fig. 1, seen from above. The mounting pad has an adhesive surface, said surface being provided with a release layer, said release layer being divided into three sections, a first section having a tongue (10) defining an essentially central axis of the mounting pad and two projecting parts (11, 12) on each side of the tongue (10). As the first section is placed between the adhesive surface of the mounting pad and a second and a third section only the projecting parts of it can be seen from this view-point. Accordingly, the first section of the release layer is sandwiched between a part of the mounting pad substantially covering the body part of the first section of the release layer, and the corresponding parts of the second and third section of the release layer. The layered structure of the mounting pad is thus in 3 layers; primary layer being the mounting pad; secondary layer being the first section of the release layer and the tertiary layer being partly the second section and partly the third section of the release layer. The second section comprises one projecting part (8) and a third section comprises one projecting part (8'). The second and third sections can as well only be seen by their projecting parts (11, 12) from this view-point. The second and third sections are separated along the central axis and have their projecting parts (8, 8') arranged on top of the projecting parts (11, 12) of the first section. The projecting parts (8, 8') have holes (16, 17) for attaching the same to the housing of an injector device, e.g. using buttons, clips or other securing means. In fig. 8 it can be seen that besides the firstsection of the second part of the release layer is divided into at least two parts, a second (20) and a third (21) section of the release layer. The second and third sections of the release layer protect the largest part of the adhesive surface of the mounting pad, which firmly secures the mounting pad to the skin of a patient.

The mounting pad has a hole for providing access e.g. for a cannula and/or a needle. This hole can have various shapes and forms.

Three projecting parts of the release layer (10, 8, 8') provide means for securing the release layer to a housing of an injector device. The central projecting part (10) is a part of or connected to the first section of the release layer, while the other two projecting parts (8, 8') are parts of or connected to the second and third sections of the release layer. In the shown embodiment, the central projecting part (10) is adapted to be secured to one part of the housing, preferably a pivoting member while the other two projecting parts (8, 8') will be secured to a different part of the housing. The end of the central projecting part (10) has an enlarged surface area (15) for securing the projecting part (10) to the pivoting member (36) of an injector device.

This embodiment of the mounting pad functions accordingly when it is a part of the adhesive device according to the present invention. The injector device including the infusion part on the mounting pad is placed in close contact with the skin of the user. A pivoting member on the injector device is pulled back in order to unlock the device and to prepare the application of the infusion set, and subsequently the injector device is actuated and the infusion part is applied on the skin of the patient. As the central projecting part (10) is attached to the pivoting member and the other two projecting parts (8, 8') of the second and third sections are secured to the other side of the housing, when the injector device is actuated, the central projecting part (10) pulls a part of the release layer in one direction while the other two projecting parts (8, 8') pull in the opposite direction, thereby peeling the release layer off the mounting pad and exposing the adhesive surface underneath. The second and third sections remain attached to the housing while the first section can be undone from the pivoting member. Finally the first section of the release layer is removed manually by pulling the central projecting part (10).

Fig. 8 shows a top view of the mounting pad according to the invention. The embodiment is the same as described in fig. 7 but viewed from below. The central projecting part (10), or a tongue projection, is a part of or connected to a first section of the release layer, which can not seen from this view-point, while the other two projecting parts (8, 8') are parts of the second (20) and third (21) section of the release layer. The second (20) and third (21) sections of the release layer essentially cover a larger part of the adhesive layer than the first release layer. By separating the release layer into the second (20) and third (21) sections along the central axis of the mounting pad, it is made possible to quickly and automatically remove the release layer without influencing the movement of the needle during insertion, and avoiding oblique forces acting on the cannula.

Fig. 9 shows one embodiment of the first section (19) of the release layer which can be used in the embodiments shown in figs. 2, 3, 5 and 6.

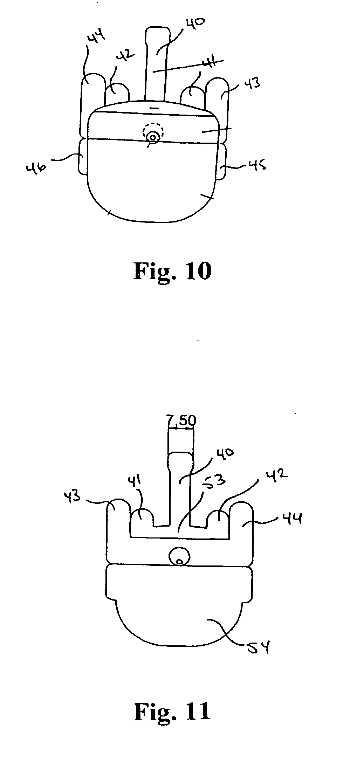

Fig. 10 shows another example of the mounting pad according to the present invention, viewed from above. This embodiment is suitable for manual removal of the release layer. The mounting pad has an adhesive surface, said surface being provided with a release layer, said release layer being divided into three sections perpendicular to the central axis of the mounting pad, a first section (body part can not be seen from this view) having tongue (40) defining an essentially central axis of the mounting pad and two projecting parts (41, 42) on each side of the tongue (40), a second section (can not be seen) comprising two projecting parts (43, 44) and a third section (can not be seen from this view) comprising two projecting parts (45, 46). The projecting parts (41, 42) of the first section are adjacent to the tongue (40) and the projecting parts (43, 44) of the second section are adjacent to the two projecting parts (41, 42) of the first section when the sections are arranged together. The third section comprises two minor projecting parts (45, 46) to facilitate the removal of the third section of the release layer. From this view only the projecting parts and tongue can be seen.

This example of the mounting pad functions accordingly when it is a part of the adhesive device according to the present invention. The injector device including the infusion part on the mounting pad is placed in close contact with the skin of the user. A pivoting member on the injector device is pulled back in order to unlock the device and to prepare the application of the infusion set. When the pivoting member is pulled back the second section of the release layer is accessible and subsequently removed by pulling one of the projecting parts (43, 44) thereby exposing the adhesive surface underneath. The injector device is actuated and the infusion part is applied on the skin of the patient. The tongue (40) is thereafter manually released from the pivoting member. To facilitate the removal of the second section of the release layer the user applies pressure with his/her fingers on projecting parts (41, 42) while removing the third section of the release layer by pulling one of projecting parts (45, 46) and finally the first section of the release layer is removed manually by pulling the tongue (40).

Fig. 11 shows the same example of the mounting pad as shown in fig. 5 (10), viewed from below. In this figure it is apparent that the outermost projecting parts (43, 44) are a part of the second section (52) of the release layer. Accordingly, this second section (52) can be removed separately from the first (53) and third (54) sections, e.g. prior to actuation of the injector device or when the injector device is actuated.

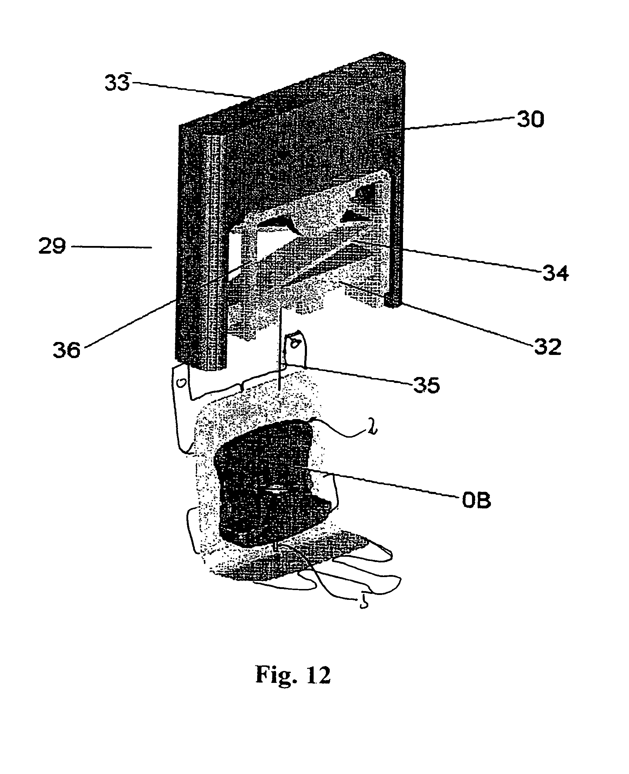

Fig. 12 shows an example of an injector device (29) according to the present invention in which the injector device and an infusion part (0B) provided with a mounting pad (2) are shown in an exploded view. The injector device comprises a housing (30) having a back (33) and being provided with two longitudinally extending guiding means (31) and a longitudinally slidable member (32). The device further comprises a pivoting member (36) which may engage with means for locking the pivoting member in one position where it protects the a cannula (5) of the infusion part (0B) to be introduced into a patient's skin and a needle (35) for introducing the cannula. The slidable member is capable of moving from a retracted position to a forward position, and is driven from the retracted position to the forward position by a spring (34). The spring is located between the slidable member (32) and the back (33) of the housing (30). Optionally there is a spring support which fits with the back of the housing thereby minimizing the risk of a malfunctioning spring. The injector device further comprises locking means for (not shown) for locking the slidable member (32) in the retracted position for maintaining the spring in a compressed state and release means (not shown) for disengaging the locking means. When the locking means is disengaged, the spring (34) drives the slidable member (32) and the infusion part (0B) to its forward position, thus introducing the cannula (5) of the infusion part into the patient by means of the needle (35). After the introduction of the cannula the injector device can be withdrawn leaving the needle in a free position, and the infusion part is secured to the skin using the mounting pad (2). The pivoting member (36) can then be swung into a position in which it embraces the needle and protects patients or assisting persons from being stung.

Furthermore, the figure also schematically shows the release layer of the kind shown in figs. 7-9.

## Claims

1. An adhesive device comprising an application device having a housing (30) and a mounting pad (2) having an adhesive surface, said surface being provided with a release layer (9), **characterized in that** the peeling of the release layer off the mounting pad is effected automatically, when in use the mounting pad is displaced in relation to the application device as the release layer (9) is secured to the housing (30).

2. An adhesive device according to claim 1, wherein the mounting pad (2) is longitudinally displaceable in relation to the housing (30) of the application device by which displacement a spring (34) is set, said housing (30) comprising means for retaining the mounting pad (2) in that position, said device further comprising means (39) for actuating the spring releasing the mounting pad (2).

3. An adhesive device according to any one of the claims 1 to 2, wherein the release layer is divided into two or more sections.

4. An adhesive device according to any one of claims 1 to 3, wherein at least one projecting part (11, 12, 8, 8', 41, 42, 43, 44) of the first (19, 53), second (20, 52) or third (21, 54) section is secured to the housing of the application device (30).

5. An adhesive device according to any one of claims 1 to 4, wherein the release layer provides a non-touch feature.

6. An adhesive device according to any of the claims 1 to 5, wherein said release layer (9) is having at least three projecting parts (7, 10, 8, 8', 40, 41, 42, 43, 44) where a first projecting part (7, 10, 40) defines an essentially central axis of the mounting pad.

7. An adhesive device according to claim 6, wherein the first projecting part (7, 10, 40) is projecting longer than the other at least two projecting parts and the projecting parts (7, 10, 8, 8', 40, 41, 42, 43, 44) are essentially parallel to each other.

8. An adhesive device according to claim 6 or 7, wherein the release layer is divided into two or more sections (19, 20, 21, 52, 53, 54).

9. An adhesive device according to any of the claims 6 to 8, wherein the release layer is divided into at least two sections (20, 21, 52, 53, 54), a first section (19, 52) being provided with a first projecting part (7, 10, 40) and two projecting parts (11, 12 , 41, 42), one on each side of the first projecting part (7, 10, 40) and a remaining section (20, 21, 52, 54) being provided with two projecting parts (8, 8', 43, 44).

10. An adhesive device according to any one of claims 6 to 9, wherein the two projecting parts (8, 8') of the remaining sections (20, 21) overlap with the two projecting parts (11, 12) of the first section (19).

11. An adhesive device according to any one of claims 6 to 10, wherein the remaining section of the release layer is divided into two sections (20, 21, 52, 54).

12. An adhesive device according to claim 11, wherein the remaining section (20, 21, 52, 54) is divided along the central axis of the mounting pad.

13. An adhesive device according to claim 11, wherein the remaining section (52, 54) is divided perpendicular to the central axis of the mounting pad.

14. An adhesive device according to any one of claims 6 to 13, wherein the sections (20, 21, 30, 52, 53, 54) of the release layer can be removed from the mounting pad independently from each other.

15. An adhesive device according to any one of claims 6 to 14, wherein the adhesive component is a skin friendly adhesive provided with a microporous backing.

16. An adhesive device according to any one of claims 6 to 15, wherein the release layer is made of siliconized paper.

17. An adhesive device according to any one of claims 6 to 16, having an adhesive surface, said surface being provided with a release layer, said release layer being divided into three sections perpendicularly to the central axis of the mounting pad, a first section (53) having a tongue (40) projecting along an essentially central axis of the mounting pad and two projecting parts (41, 42) on each side of the tongue (40), a second section (52) comprising two projecting parts (43, 44) and a third section (54), wherein the projecting parts (41, 42) of the first section (53) essentially parallel to and spaced apart from the tongue (40) and the projecting parts (43, 44) of the second section (52) are superposed on the two projecting parts (41, 42) of the first section (53) when the sections are arranged together on the adhesive surface.

18. An adhesive device according to any one of claims 6 to 17, having an adhesive surface, said surface being provided with a release layer, said release layer being divided into three sections, a first section having a tongue (10, 7) projecting along an essentially central axis of the mounting pad and two projecting parts (11, 12) on each side of the tongue, said first section being placed on the front part of the adhesive surface between the front part of the adhesive surface and the second (20) and third (21) sections, the second (20) and third (21) sections each comprising one projecting part (8, 8'), said second (20) and third (21) sections being separated along the central axis of the mounting pad, and the projecting parts (8, 8') of the second (20) and third (21) sections of the release layer being superposed on the projecting parts (11, 12) of the first section.

19. An adhesive device according to any of the claims 1 to 18, comprising an injector device for the subcutaneous introduction of a cannula (5) of an infusion part (0B) of an infusion set into the skin of a patient, said infusion part comprising a mounting pad (2) having an adhesive component having an adhesive surface, said surface being provided with a release layer (9), said release layer (9) having at least three (7, 10, 8, 8', 40, 41, 42, 43, 44) projecting parts where a first projecting part (7, 10, 40) is projecting along an essentially central axis of the mounting pad (2), said device comprising a housing (30), a back (33) and longitudinally extending guiding means (31), a member (32) which is longitudinally slidable within the housing (30) and comprising an insertion needle (35), a spring (34) located between the back of the housing and the longitudinally slidable member (32), locking means (38) for maintaining the spring in a compressed state and release means (39) for disengaging the locking means (38), said device further comprising a pivoting member (36) which can be swung into a position in which it embraces the needle, wherein a projecting part (8, 8') of the release layer (9) is secured to the housing (30) of the injector device.

20. An adhesive device according to claim 19, comprising an injector device for the subcutaneous introduction of a cannula (5) of an infusion part (0B) of an infusion set into the skin of a patient, said infusion part comprising a mounting pad (2) having an adhesive component having an adhesive surface, said surface being provided with a release layer (9), said release layer (9) having at least three (7, 10, 8, 8', 40, 41, 42, 43, 44) projecting parts one of which is (7, 10, 40) projecting along an essentially central axis of the mounting pad (2), said device comprising a housing (30), a back (33) and longitudinally extending guiding means (31), a member (32) which is longitudinally slidable within the housing (30) and comprising a needle (35) for insertion in the cavity of said cannula, a spring (34) located between the back of the housing and the longitudinally slidable member (32), locking means for maintaining the spring in a compressed state and release means (39) for disengaging the locking means, said device further comprising a pivoting member (36) which can be swung into a position in which it is placed parallel to the housing (30) into a position in which it embraces the needle, wherein a projecting part (8, 8') of the release layer is secured to the housing (30) of the injector device.

21. An adhesive device according to claim 19 or 20, comprising an injector device **characterized in that** the pivoting member (36) has fixing means for temporarily fixing a projecting part of the release layer (9) to the pivoting member (36).

22. An adhesive device according to claim 19 or 20, wherein the first cannula (5) extending from said base part is being in fluid communication with a cavity which is optionally covered with a membrane, said cavity being adapted to receive a second cannula extending from a connector.

23. A method for preparing an infusion for application, comprising providing an infusion part comprising an adhesive device comprising an application device having a housing (30) and a mounting pad (2) having an adhesive component having an adhesive surface, said surface being provided with a release layer (9), said release layer (9) having at least three (7, 8, 8', 10, 11, 12, 40, 41, 42, 43, 44) projecting parts where a first projecting part (7, 10, 40) is projecting along an essentially central axis of the mounting pad (2) and an injector device, said injector device comprising a housing (30), a back (33) and longitudinally extending guiding means (31), a member (32) which is longitudinally slidable within the housing (30) and comprising a needle (35) for insertion in the cavity of a cannula, a spring (34) located between the back (33) of the housing (30) and the longitudinally slidable member (32), locking means for maintaining the spring in a compressed state and release means (39) for disengaging the locking means, said device further comprising a pivoting member (36) which can be swung into a position in which it embraces the needle (35),
placing the infusion part (0B) in the injector device,
setting the spring (34) of the injector device, and
securing or connecting a projecting part (7, 10, 40) of the release layer (9) to the housing (30) of the injector device.

24. A method for preparing an infusion set for application, comprising providing an infusion part comprising an adhesive device comprising an application device having a housing (30) and a mounting pad (2) having an adhesive component having an adhesive surface, said surface being provided with a release layer, said release layer being divided into three sections and having at least three (7, 8, 8', 10, 11, 12, 40, 41, 42, 43, 44) projecting parts one of which is a tongue (7, 10, 40) projecting along an essentially central axis of the mounting pad and an injector device, said injector device comprising a housing (30), a back (33) and longitudinally extending guiding means (31), a member (32) which is longitudinally slidable within the housing (30) and comprising a needle (35) for insertion in the cavity of a cannula, a spring (34) located between the back of the housing and the longitudinally slidable member (32), locking means for maintaining the spring (34) in a compressed state and release means (39) for disengaging the locking means, said device further comprising a pivoting member (36) which can be swung into a position in which it embraces the needle (35),
placing the infusion part (0B) in the injector device,
setting the spring (34) of the injector device, and
removing a second section (52) of the release layer.

25. A kit comprising an adhesive device according to any of claims 1-22, in combination with an infusion part and an injector device.

26. An adhesive device according to claim 1, wherein the projecting parts (7, 10, 8, 8', 40, 41, 42, 43, 44) comprising a first projecting part (7, 10, 40) defining a central axis of the mounting pad and at least two secondary projecting parts are essentially parallel (8, 8', 41, 42, 43, 44).

27. An adhesive device according to claim 26, wherein the secondary projecting parts (8, 8', 41, 42, 43, 44) are positioned symmetrical around the central axis defined by the first projecting part.

## Patentansprüche

1. Klebevorrichtung, umfassend eine Applikationsvorrichtung, die ein Gehäuse (30) und ein Anbringkissen (2) aufweist, aufweisend eine Klebefläche, wobei die Fläche mit einer Freigabeschicht (9) versehen ist,
**dadurch gekennzeichnet, dass** die Schälung der Freigabeschicht des Anbringkissens automatisch erfolgt, wenn bei Verwendung das Anbringkissen im Verhältnis zur Applikationsvorrichtung verschoben ist, da die Freigabeschicht (9) am Gehäuse (30) gesichert ist.

2. Klebevorrichtung nach Anspruch 1, wobei das Anbringkissen (2) im Verhältnis zum Gehäuse (30) der Applikationsvorrichtung längs verschiebbar ist, bei dessen Verschiebung eine Feder (34) eingestellt ist, wobei das Gehäuse (30) Mittel zur Festhaltung des Anbringkissens (2) in dieser Position umfasst, welche Vorrichtung zusätzlich Mittel (39) zur Betätigung der das Anbringkissen (2) freigebenden Feder umfasst.

3. Klebevorrichtung nach irgendeinem der vorhergehenden Ansprüche 1 bis 2, wobei die Freigabeschicht in zwei oder mehrere Sektionen aufgeteilt ist.

4. Klebevorrichtung nach irgendeinem der vorhergehenden Ansprüche 1 bis 3, wobei zumindest ein vorstehender Teil (11, 12, 8, 8', 41, 42 43, 44) der ersten (19, 53), zweiten (20, 52) oder dritten (21, 54) Sektion am Gehäuse der Applikationsvorrichtung (30) gesichert ist.

5. Klebevorrichtung nach irgendeinem der vorhergehenden Ansprüche 1 bis 4, wobei die Freigabeschicht eine berührungslose Eigenschaft bereitstellt.

6. Klebevorrichtung nach irgendeinem der Ansprüche 1 bis 5, wobei die Freigabeschicht (9) mindestens drei vorstehende Teile (7, 10, 8, 8', 40, 41, 42, 43, 44) aufweist, wobei ein erster vorstehender Teil (7, 10, 40) eine im Wesentlichen mittlere Achse des Anbringkissens definiert.

7. Klebevorrichtung nach Anspruch 6, wobei der erste vorstehende Teil (7, 10, 40) länger vorsteht als die anderen zumindest beiden vorstehenden Teile, und die vorstehenden Teile (7, 10, 8, 8', 40, 41, 42, 43, 44) im Wesentlichen parallel zueinander sind.

8. Klebevorrichtung nach Anspruch 6 oder 7, wobei die Freigabeschicht in zwei oder mehrere Sektionen (19, 20,21,52,53,54) aufgeteilt ist.

9. Klebevorrichtung nach irgendeinem der Ansprüche 6 bis 8, wobei die Freigabeschicht in zumindest zwei Sektionen (20, 21, 52, 53, 54) aufgeteilt ist, wobei eine erste Sektion (19, 52) mit einem ersten vorstehenden Teil (7, 10, 40) und zwei vorstehenden Teilen (11, 12, 41, 42), einem auf jeder Seite des ersten vorstehenden Teils (7, 10, 40), versehen ist, und eine verbleibende Sektion (20, 21, 52, 54) mit zwei vorstehenden Teilen (8, 8', 43, 44) versehen ist.

10. Klebevorrichtung nach irgendeinem der Ansprüche 6 bis 9, wobei die beiden vorstehenden Teile (8, 8') der verbleibenden Sektionen (20, 21) mit den beiden vorstehenden Teilen (11, 12) der ersten Sektion (19) überlappen.

11. Klebevorrichtung nach irgendeinem der Ansprüche 6 bis 10, wobei die verbleibende Sektion der Freigabeschicht in zwei Sektionen (20, 21, 52, 54) aufgeteilt ist.

12. Klebevorrichtung nach Anspruch 11, wobei die verbleibende Sektion (20, 21, 52, 54) entlang der mittleren Achse des Anbringkissens aufgeteilt ist.

13. Klebevorrichtung nach Anspruch 11, wobei die verbleibende Sektion (52, 54) senkrecht zur mittleren Achse des Anbringkissens aufgeteilt ist.

14. Klebevorrichtung nach irgendeinem der Ansprüche 6 bis 13, wobei die Sektionen (20, 21, 30, 52, 53, 54) der Freigabeschicht unabhängig voneinander aus dem Anbringkissen entfernbar sind.

15. Klebevorrichtung nach irgendeinem der Ansprüche 6 bis 14, wobei die Klebekomponente ein mit einer mikroporösen Deckung versehener hautfreundlicher Kleber ist.

16. Klebevorrichtung nach irgendeinem der Ansprüche 6 bis 15, wobei die Freigabeschicht aus silikonisiertem Papier hergestellt ist.

17. Klebevorrichtung nach irgendeinem der Ansprüche 6 bis 16, aufweisend eine Klebefläche, welche Fläche mit einer Freigabeschicht versehen ist, welche Freigabeschicht in drei zur mittleren Achse des Anbringkissens senkrechte Sektionen aufgeteilt ist, eine erste Sektion (53), die eine entlang einer im Wesentlichen mittleren Achse des Anbringkissens vorstehende Zunge (40) und zwei vorstehende Teile (41, 42) auf jeder Seite der Zunge (40) aufweist, eine zwei vorstehende Teile (43, 44) umfassende zweite Sektion (52) und eine dritte Sektion (54), wobei die vorstehenden Teile (41, 42) der ersten Sektion (53) im Wesentlichen parallel zur und von der Zunge (40) beabstandet sind, und die vorstehenden Teile (43, 44) der zweiten Sektion (52) auf den beiden vorstehenden Teilen (41, 42) der ersten Sektion (53) angelegt sind, wenn die Sektionen gemeinsam auf der Klebefläche angebracht sind.

18. Klebevorrichtung nach irgendeinem der Ansprüche 6 bis 17, aufweisend eine Klebefläche, welche Fläche mit einer Freigabeschicht versehen ist, welche Freigabeschicht in drei Sektionen aufgeteilt ist, eine erste Sektion, die eine entlang einer im Wesentlichen mittleren Achse des Anbringkissens vorstehende Zunge (10, 7) und zwei vorstehende Teile (11, 12) auf jeder Seite der Zunge aufweist, welche erste Sektion am vorderen Teil der Klebefläche zwischen dem vorderen Teil der Klebefläche und der zweiten (20) und dritten (21) Sektion angebracht ist, wobei die zweite (20) und dritte (21) Sektion jeweils einen vorstehenden Teil (8,8') umfassen, welche zweite (20) und dritte (21) Sektion entlang der mittleren Achse des Anbringkissens getrennt sind, und die vorstehenden Teile (8, 8') der zweiten (20) und dritten (21) Sektion der Freigabeschicht auf den vorstehenden Teilen (11, 12) der ersten Sektion angelegt sind.

19. Klebevorrichtung nach irgendeinem der Ansprüche 1 bis 18, umfassend eine Injektorvorrichtung zur subkutanen Einführung einer Kanüle (5) eines Infusionsteils (OB) eines Infusionssets in die Haut eines Patienten, welcher Infusionsteil ein Anbringkissen (2) umfasst, das eine eine Klebefläche aufweisende Klebekomponente aufweist, welche Fläche mit einer Freigabeschicht (9) versehen ist, welche Freigabeschicht (9) mindestens drei (7, 10, 8, 8' 40, 41, 42, 43, 44) vorstehende Teile aufweist, wobei ein erster vorstehender Teil (7, 10, 40) entlang einer im Wesentlichen mittleren Achse des Anbringkissens (2) vorsteht, welche Vorrichtung ein Gehäuse (30), einen Hinterteil (33) und längs erstreckende Führungsmittel (31), ein innerhalb des Gehäuses (30) längs verschiebbares und eine Einführnadel (35) umfassendes Element (32), eine zwischen dem Hinterteil des Gehäuses und dem längs verschiebbaren Element (32) befindliche Feder (34), Verriegelungsmittel (38) zur Beibehaltung der Feder in einem komprimierten Zustand und Freigabemittel (39) zur Entlastung der Verriegelungsmittel (38) umfasst, welche Vorrichtung zusätzlich ein in eine Position schwenkbares drehbares Element (36) umfasst, in der es die Nadel umschließt, wobei ein vorstehender Teil (8, 8') der Freigabeschicht (9) am Gehäuse (30) der Injektorvorrichtung gesichert ist.

20. Klebevorrichtung nach Anspruch 19, umfassend eine Injektorvorrichtung zur subkutanen Einführung einer Kanüle (5) eines Infusionsteils (OB) eines Infusionssets in die Haut eines Patienten, welcher Infusionsteil ein Anbringkissen (2) umfasst, das eine eine Klebefläche aufweisende Klebekomponente aufweist, welche Fläche mit einer Freigabeschicht (9) versehen ist, welche Freigabeschicht (9) mindestens drei (7, 10, 8, 8' 40, 41, 42, 43, 44) vorstehende Teile aufweist, von denen einer (7, 10, 40) entlang einer im Wesentlichen mittleren Achse des Anbringkissens (2) vorsteht, welche Vorrichtung ein Gehäuse (30), einen Hinterteil (33) und längs erstreckende Führungsmittel (31), ein innerhalb des Gehäuses (30) längs verschiebbares und eine Nadel (35) zur Einführung in den Hohlraum der Kanüle umfassendes Element (32), eine zwischen dem Hinterteil des Gehäuses und dem längs verschiebbaren Element (32) befindliche Feder (34), Verriegelungsmittel zur Beibehaltung der Feder in einem komprimierten Zustand und Freigabemittel (39) zur Entlastung der Verriegelungsmittel umfasst, welche Vorrichtung zusätzlich ein in eine Position schwenkbares drehbares Element (36) umfasst, in der es parallel zum Gehäuse (30) in einer Position angebracht ist, in der es die Nadel umschließt, wobei ein vorstehender Teil (8, 8') der Freigabeschicht am Gehäuse (30) der Injektorvorrichtung gesichert ist.

21. Klebevorrichtung nach Anspruch 19 oder 20, umfassend eine Injektorvorrichtung, **dadurch gekennzeichnet, dass** das drehbare Element (36) Fixierungsmittel zum vorübergehenden Fixieren eines vorstehenden Teils der Freigabeschicht (9) am drehbaren Element (36) aufweist.

22. Klebevorrichtung nach Anspruch 19 oder 20, wobei sich die erste vom Basisteil erstreckende Kanüle (5) mit einem mit einer Membran wahlweise gedeckten Hohlraum in Fluidverbindung steht, welcher Hohlraum zur Aufnahme einer zweiten sich von einem Verbinder erstreckenden Kanüle ausgelegt ist.

23. Verfahren zum Vorbereiten einer Infusion zur Applikation, umfassend das Bereitstellen eines Infusionsteils, umfassend eine Klebevorrichtung, umfassend eine ein Gehäuse (30) und ein eine Klebekomponente aufweisendes Anbringkissen (2) aufweisende Applikationsvorrichtung, welche Klebekomponente eine Klebefläche aufweist, welche Fläche mit einer Freigabeschicht (9), welche Freigabeschicht (9) mindestens drei (7, 8, 8', 10, 11, 12, 40, 41, 42, 43, 44) vorstehende Teile aufweist, wobei ein erster vorstehender Teil (7, 10, 40) entlang einer im Wesentlichen mittleren Achse des Anbringkissens (2) vorsteht, und einer Injektorvorrichtung versehen wird, welche Injektorvorrichtung ein Gehäuse (30), einen Hinterteil (33) und längs vorstehende Führungsmittel (31), ein innerhalb des Gehäuses (30) längs verschiebbares und eine Nadel (35) zur Einführung in den Hohlraum einer Kanüle umfassendes Element (32), eine zwischen dem Hinterteil (33) des Gehäuses (30) und dem längs verschiebbaren Element (32) befindliche Feder (34), Verriegelungsmittel zur Beibehaltung der Feder in einem komprimierten Zustand und Freigabemittel (39) zur Entlastung der Verriegelungsmittel umfasst, welche Vorrichtung zusätzlich ein in eine Position schwenkbares drehbares Element (36) umfasst, in der es die Nadel (35) umschließt,
das Anbringen des Infusionsteils (OB) in der Injektorvorrichtung,
das Einstellen der Feder (34) der Injektorvorrichtung, und
die Sicherung oder Verbindung eines vorstehenden Teils (7, 10, 40) der Freigabeschicht (9) an/mit dem Gehäuse (30) der Injektorvorrichtung.

24. Verfahren zum Vorbereiten eines Infusionssets zur Applikation, umfassend
das Bereitstellen eines Infusionsteils, umfassend eine Klebevorrichtung, umfassend eine ein Gehäuse (30) und ein eine Klebekomponente aufweisendes Anbringkissen (2) aufweisende Applikationsvorrichtung, welche Klebekomponente eine Klebefläche aufweist, welche Fläche mit einer Freigabeschicht, welche Freigabeschicht in drei Sektionen aufgeteilt wird und mindestens drei (7, 8, 8', 10, 11, 12, 40, 41, 42, 43, 44) vorstehende Teile aufweist, von denen einer eine entlang einer im Wesentlichen mittleren Achse des Anbringkissens vorstehende Zunge (7, 10, 40) ist, und einer Injektorvorrichtung versehen wird, welche Injektorvorrichtung ein Gehäuse (30), einen Hinterteil (33) und längs vorstehende Führungsmittel (31), ein innerhalb des Gehäuses (30) längs verschiebbares und eine Nadel (35) zur Einführung in den Hohlraum einer Kanüle umfassendes Element (32), eine zwischen dem Hinterteil des Gehäuses und dem längs verschiebbaren Element (32) befindliche Feder (34), Verriegelungsmittel zur Beibehaltung der Feder (34) in einem komprimierten Zustand und Freigabemittel (39) zur Entlastung der Verriegelungsmittel umfasst, welche Vorrichtung zusätzlich ein in eine Position schwenkbares drehbares Element (36) umfasst, in der es die Nadel (35) umschließt, das Anbringen des Infusionsteils (OB) in der Injektorvorrichtung,
das Einstellen der Feder (34) der Injektorvorrichtung, und
das Entfernen einer zweiten Sektion (52) der Freigabeschicht.

25. Ausstattung, umfassend eine Klebevorrichtung nach irgendeinem der Ansprüche 1-22 in Kombination mit einem Infusionsteil und einer Injektorvorrichtung.

26. Klebevorrichtung nach Anspruch 1, wobei die vorstehenden Teile (7, 10, 8, 8',40, 41, 42, 43, 44) einen eine mittlere Achse des Anbringkissens definierenden ersten vorstehenden Teil (7, 10, 40) umfasst, und wenigstens zwei sekundäre vorstehende Teile im Wesentlichen parallel (8, 8', 41, 42, 43, 44) sind.

27. Klebevorrichtung nach Anspruch 26, wobei die sekundären vorstehenden Teile (8, 8', 41, 42, 43, 44) symmetrisch um die durch den ersten vorstehenden Teil definierte mittlere Achse positioniert sind.

## Revendications

1. Dispositif adhésif comprenant un dispositif d'application présentant un boîtier (30) et un tampon de montage (2) avec une surface adhésive, ladite surface étant pourvue d'une couche de libération (9), **caractérisé en ce que** le détachement de la couche de libération du tampon de montage est effectué automatiquement si lors de l'usage, le tampon de montage est déplacé par rapport au dispositif d'application quand la couche de libération (9) est fixée au boîtier (30).

2. Dispositif adhésif selon la revendication 1, dans lequel le tampon de montage (2) peut se déplacer longitudinalement par rapport au boîtier (30) du dispositif d'application, déplacement par lequel un ressort (34) est tendu, ledit boîtier (30) comprenant des moyens pour retenir le tampon de montage (2) dans cette position, ledit dispositif comprenant en outre des moyens (39) pour activer le ressort libérant le tampon de montage (2).

3. Dispositif adhésif selon l'une quelconque des revendications 1 à 2, dans lequel la couche de libération est divisée en deux ou plusieurs sections.

4. Dispositif adhésif selon l'une quelconque des revendications 1 à 3, dans lequel au moins une partie en saillie (11, 12, 8, 8', 41, 42, 43, 44) de la première section (19, 53), de la deuxième section (20, 52) ou de la troisième section (21, 54) est fixée au boîtier du dispositif d'application (30).

5. Dispositif adhésif selon l'une quelconque des revendications 1 à 4, dans lequel la couche de libération présente une caractéristique de non-toucher.

6. Dispositif adhésif selon l'une quelconque des revendications 1 à 5, dans lequel la couche de libération (9) est pourvue d'au moins trois parties en saillie (7, 10, 8, 8', 40, 41, 42, 43, 44), où la première partie en saillie (7, 10, 40) définit un axe essentiellement central du tampon de montage.

7. Dispositif d'application selon la revendication 6, dans lequel la première partie en saillie (7, 10, 40) fait une plus grande saillie que les autres au moins deux parties en saillie, et les parties en saillie (7, 10, 8, 8', 40, 41, 42, 43, 44) sont essentiellement parallèles l'une à l'autre.

8. Dispositif adhésif selon la revendication 6 ou 7, dans lequel la couche de libération est divisée en deux ou plusieurs sections (19, 20, 21, 52, 53, 54).

9. Dispositif adhésif selon l'une quelconque des revendications 6 à 8, dans lequel la couche de libération est divisée en au moins deux sections (20, 21, 52, 53, 54), une première section (19, 52) étant pourvue d'une première partie en saillie (7, 10, 40) et deux parties en saillie (11, 12, 41, 42) dont l'une sur chaque côté de la première partie en saillie (7, 10, 40), et une section restante (20, 21, 52, 54) étant pourvue de deux parties en saillie (8, 8', 43, 44).

10. Dispositif adhésif selon l'une quelconque des revendications 6 à 9, dans lequel les deux parties en saillie (8, 8') des sections restantes (20, 21) recouvrent les deux parties en saillie (11, 12) de la première section (19).

11. Dispositif adhésif selon l'une quelconque des revendications 6 à 10, dans lequel la section restante de la couche de libération est divisée en deux sections (20, 21, 52, 54).

12. Dispositif adhésif selon la revendication 11, dans lequel la section restante (20, 21, 52, 54) est divisée le long de l'axe central du tampon de montage.

13. Dispositif adhésif selon la revendication 11, dans lequel la section restante (52, 54) est divisée perpendiculairement à l'axe central du tampon de montage.

14. Dispositif adhésif selon l'une quelconque des revendications 6 à 13, dans lequel les sections (20, 21, 30, 52, 53, 54) de la couche de libération peuvent être détachées du tampon de montage, l'une indépendante de l'autre.

15. Dispositif d'adhésif selon l'une quelconque des revendications 6 à 14, dans lequel l'élément adhésif est une colle agréable sur la peau et pourvue d'une face postérieure microporeuse.

16. Dispositif adhésif selon l'une quelconque des revendications 6 à 15, dans lequel la couche de libération est réalisée du papier siliconé.

17. Dispositif adhésif selon l'une quelconque des revendications 6 à 16, présentant une surface adhésive, ladite surface étant pourvue d'une couche de libération, ladite couche de libération étant divisée en trois sections arrangées perpendiculairement à l'axe central du tampon de montage, une première section (53) présentant une languette (40) faisant saillie le long d'un axe essentiellement central du tampon de montage et deux parties en saillie (41, 42) sur chaque côté de la languette (40), une deuxième section (52) comprenant deux parties en saillie (43, 44) et une troisième section (54), où les sections en saillie (41, 42) de la première section (53) essentiellement parallèle à et espacée de la languette (40) et les parties en saillie (43, 44) de la deuxième section (52) sont superposées sur les deux parties en saillie (41, 42) de la première section (53) quand les sections sont arrangées l'une à côté de l'autre sur la surface adhésive.

18. Dispositif adhésif selon l'une quelconque des revendications 6 à 17, présentant une surface adhésive, ladite surface étant pourvue d'une couche de libération, ladite couche de libération étant divisée en trois sections, une première section présentant une languette (10, 7) faisant saillie le long d'un axe essentiellement central du tampon de montage et deux parties en saillie (11, 12) sur chaque côté de la languette (40), ladite première section étant arrangée sur la partie antérieure de la surface adhésive entre la partie antérieure de la surface adhésive et la deuxième section (20) et la troisième section (21), la deuxième section (20) et la troisième section (21) chacune comprenant une partie en saillie (8, 8'), ladite deuxième section (20) et ladite troisième section (21) étant séparées le long de l'axe central du tampon de montage, et les parties en saillie (8, 8') de la deuxième section (20) et de la troisième section (21) de la couche de libération étant superposées sur les parties en saillie (11, 12) de la première section.

19. Dispositif adhésif selon l'une quelconque des revendications 1 à 18, comprenant un dispositif d'injection pour l'introduction sous-cutanée d'une canule (5) d'un élément d'infusion (0B) d'un perfuseur dans la peau d'un patient, ledit élément d'infusion comprenant un tampon de montage (2) présentant un élément adhésif avec une surface adhésive, ladite surface étant pourvue d'un couche de libération (9), ladite couche de libération (9) présentant au moins trois parties en saillie (7, 10, 8, 8', 40, 41, 42, 43, 44), où une première partie en saillie (7, 10, 40) fait saillie le long d'un axe essentiellement central du tampon de montage (2), ledit dispositif comprenant un boîtier (30), un dos (33) et des moyens de guidage (31) s'étendant longitudinalement, un organe (32) qui peut glisser longitudinalement dans le boîtier (30) et comprend une aiguille d'insertion (35), un ressort (34) arrangé entre le dos du boîtier et l'organe (32) susceptible de glisser longitudinalement, des moyens de verrouillage (38) pour maintenir le ressort dans un état compressé et des moyens de libération (39) pour désengager les moyens de verrouillage (38), ledit dispositif comprenant en outre un élément pivotant (36) qui peut être pivoté dans un position dans laquelle il enveloppe l'aiguille, où une partie en saillie (8, 8') de la couche de libération (9) est fixée au boîtier (30) du dispositif d'injection.

20. Dispositif adhésif selon la revendication 19, comprenant un dispositif d'injection pour l'introduction sous-cutanée d'une canule (5) d'un élément d'infusion (0B d'un perfuseur dans la peau d'un patient, ledit élément d'infusion comprenant un tampon de montage (2) présentant un élément adhésif avec une surface adhésive, ladite surface étant pourvue d'un couche de libération (9), ladite couche de libération (9) présentant au moins trois parties en saillie (7, 10, 8, 8', 40, 41, 42, 43, 44), dont l'une (7, 10, 40) fait saillie le long d'un axe essentiellement central du tampon de montage (2), ledit dispositif comprenant un boîtier (30), un dos (33) et des moyens de guidage (31) s'étendant longitudinalement, un organe (32) qui peut glisser longitudinalement dans le boîtier (30) et comprend une aiguille (35) pour l'insertion dans la cavité de ladite canule, un ressort (34) arrangé entre le dos du boîtier et l'organe (32) susceptible de glisser longitudinalement, des moyens de verrouillage pour maintenir le ressort dans un état compressé et des moyens de libération (39) pour désengager les moyens de verrouillage, ledit dispositif comprenant en outre un élément pivotant (36) qui peut être pivoté dans un position dans laquelle il est situé parallèlement au boîtier (30) dans une position dans laquelle il enveloppe l'aiguille, où une partie en saillie (8, 8') de la couche de libération est fixée au boîtier (30) du dispositif d'injection.

21. Dispositif adhésif selon la revendication 19 ou 20, comprenant un dispositif d'injection **caractérisé en ce que** l'élément pivotant (36) présente des moyens de fixation pour la fixation temporaire d'une partie en saillie de la couche de libération (9) à l'élément pivotant (36).

22. Dispositif adhésif selon la revendication 19 ou 20, dans lequel la première canule (5) s'étendant de ladite partie de base est en communication fluide avec une cavité qui peut éventuellement être couverte d'une membrane, ladite cavité étant adaptée à recevoir une deuxième canule s'étendant depuis un raccord.

23. Procédé pour la préparation d'une infusion à l'application, comprenant :
réalisation d'un élément d'infusion comprenant un dispositif adhésif comprenant un dispositif d'application avec un boîtier (30) et un tampon de montage (2) présentant un élément adhésif avec une surface adhésive, ladite surface étant pourvue d'un couche de libération (9), ladite couche de libération (9) présentant au moins trois parties en saillie (7, 8, 8', 10, 11, 12, 40, 41, 42, 43, 44) où une première partie en saillie (7, 10, 40) fait saillie le long d'un axe essentiellement central du tampon de montage (2), et un dispositif d'injection, ledit dispositif d'injection comprenant un boîtier (30), un dos (33) et des moyens de guidage (31) s'étendant longitudinalement, un organe (32) qui peut glisser longitudinalement dans le boîtier (30) et comprend une aiguille (35) pour l'insertion dans la cavité d'une canule, un ressort (34) arrangé entre le dos (33) du boîtier (30) et l'organe (32) susceptible de glisser longitudinalement, des moyens de verrouillage pour maintenir le ressort dans un état compressé et des moyens de libération (39) pour désengager les moyens de verrouillage, ledit dispositif comprenant en outre un élément pivotant (36) qui peut être pivoté dans un position dans laquelle il enveloppe l'aiguille (35),
mise en place de l'élément d'infusion (0B) dans le dispositif d'injection, tension du ressort (34) du dispositif d'injection, et
fixation ou couplage d'une partie en saillie (7, 10, 40) de la couche de libération (9) au boîtier (30) du dispositif d'injection.

24. Procédé pour la préparation d'un perfuseur à l'application, comprenant :
réalisation d'un élément d'infusion comprenant un dispositif adhésif comprenant un dispositif d'application avec un boîtier (30) et un tampon de montage (2) présentant un élément adhésif avec une surface adhésive, ladite surface étant pourvue d'un couche de libération, ladite couche de libération étant divisée en trois sections et présentant au moins trois parties en saillie (7, 8, 8', 10, 11, 12, 40, 41, 42, 43, 44), dont l'une est une languette (7, 10, 40) faisant saillie le long d'un axe essentiellement central du tampon de montage, et un dispositif d'injection, ledit dispositif d'injection comprenant un boîtier (30), un dos (33) et des moyens de guidage (31) s'étendant longitudinalement, un organe (32) qui peut glisser longitudinalement dans le boîtier (30) et comprend une aiguille (35) pour l'insertion dans la cavité d'une canule, un ressort (34) arrangé entre le dos du boîtier et l'organe (32) susceptible de glisser longitudinalement, des moyens de verrouillage pour maintenir le ressort (34) dans un état compressé et des moyens de libération (39) pour désengager les moyens de verrouillage, ledit dispositif comprenant en outre un élément pivotant (36) qui peut être pivoté dans un position dans laquelle il enveloppe l'aiguille (35),
mise en place de l'élément d'infusion (0B dans le dispositif d'injection, tension du ressort (34) du dispositif d'injection, et
suppression d'une deuxième section (52) de la couche de libération.

25. Trousse comprenant un dispositif adhésif selon l'une quelconque des revendications 1 à 22, en combinaison avec un élément d'infusion et un dispositif d'injection.

26. Dispositif adhésif selon la revendication 1, dans lequel les parties en saillie (7, 10, 8, 8', 40, 41, 42, 43, 44) comprenant une première partie en saillie (7, 10, 40) définissant un axe central du tampon de montage et au moins deux deuxièmes parties en saillie sont essentiellement parallèles les unes à les autres (8, 8', 41, 42, 43, 44).

27. Dispositif adhésif selon la revendication 26, dans lequel les deuxièmes parties en saillie (8, 8', 41, 42, 43, 44) sont positionnées symétriquement autour de l'axe central défini par la première partie en saillie.
